# EUROPEAN PATENT APPLICATION

(11) **EP 1 275 724 A1**
(43) Date of publication of application: **15.01.2003**
(21) Application number: 01116847.3
(22) Date of filing: 10.07.2001
(51) Int. Cl.: C12N 15/62, C07K 16/10, C07K 16/28, C07K 14/705, C07K 14/535, C07K 14/55, C12N 15/70, A61K 35/12, A61P 35/00

(54) **Bonding reagents for cell surface protein and effector cells**

(71) Applicant: Schirrmacher, Volker, Prof. Dr., 69117 Heidelberg (DE)
(72) Inventor: Schirrmacher, Volker, Prof. Dr., 69117 Heidelberg (DE)
(74) Representative: Schüssler, Andrea, Dr.

(57) **Abstract**

The present invention concerns a bonding reagent having a first bonding component for a cell surface protein and a second bonding component for a costimulatory acting molecule of an effector cell, characterised in that the first bonding component is a single chain Fᵥ fragment directed against the F protein of Newcastle Disease Virus on the cell surface of NDV infected cells or the first bonding component is a single chain Fv fragment comprising the peptide sequence of Fig. 8b.

## Description

The present invention relates to bonding reagents, expression plasmids expressing these reagents, and pharmaceutical compositions containing the bonding reagents.

It is known that in the case of active immunization, the cells used often only show weak or no immunogenicity. This is found particularly when tumor cells are employed.

Experiments were made to increase the immunogenicity of cells. As in the use of oncolysates obtained by Newcastle Disease Virus (referred to as NDV hereafter), such experiments often failed to yield satisfactory results.

In the patent application EP 95 911 308.5 the inventors presented a first approach to resolving this problem. EP 95 911 308.5 teaches a bonding reagent with a first bonding component for a cell surface protein and a second bonding component for a costimulatory acting molecule of an effector cell. EP 95 911 308.5 expressly describes the use of a Fv fragment directed against the hemagglutinin-neuraminidase molecule of NDV. This virus is paricularly useful for targetting tumor cells, since it selectively infects such tumor cells, but no other tissue.

Further, EP 95 911 308.5 describes the use of anti-CD28 fragments (amoung others) as second bonding components, in order to stimulate a close encounter of immune cells carrying CD28 or other proteins present on such cells, with those cells targeted by the first bonding component, e.g. NDV infected tumor cells.

The two kinds of bonding components are then connected together in order to produce the complete bonding reagent.

However, the bonding reagent of EP 95 911 308.5 had several disadvantages, e.g. the practical applicability of the particular bonding reagents described therein.

It is therefore an object of the present invention to provide better bonding reagents and bonding reagents easier to obtain and to handle.

This object is solved by providing the bonding reagents according to independent claims 1, 6, 9, and 23, the expression plasmids according to independent claims 15, 16, and 18, the vaccine according to independent claim 33, and the use according to claim 34. Further advantages, details and aspects of the invention become evident when studying the dependent claims, the description and the attached figures.

The invention is directed to novel, advantageous peptide sequences used in the bonding components of the invention.

In a particular aspect, the invention is directed to a bonding reagent combining two fully functional bonding components into a single peptide. Furthermore, it is directed to novel bonding reagents with multivalent bonding components and to the production of novel pharmaceutical compositions employing inventive bonding reagents.

The invention is, therefore, directed to a bonding reagent having a first bonding component for a cell surface protein and a second bonding component for a costimulatory acting molecule of an effector cell, characterised in that the first bonding component is a single chain Fᵥ fragment directed against the F protein of Newcastle Disease Virus on the cell surface of NDV infected cells or the first bonding component is a single chain Fᵥ fragment comprising the peptide sequence of Fig. 8b.

In this aspect of the invention, the expression "first bonding component" comprises any compound which may be linked to a cell surface protein, on the one hand, and to a second bonding component, on the other hand. Preferably, the compound is an antibody or a portion thereof which has a bonding domain. Such a portion is favourably a Fab', (Fab')₂, Fᵥ or (Fᵥ)₂ fragment.

The expression "cell surface protein" comprises any molecule which may be present on a cell surface. It may be a peptide or protein, for example, which originates from the cell as such, from a virus in the cell or from the expression of a DNA introduced into the cell. The cell surface protein is preferably a virus protein, namely the hemagglutinin-neuraminidase of the F protein of NDV, or the hemagglutinin molecule of influenza virus, or the coat protein of HTLV-1 or HIV. The cell surface protein may further be a growth factor receptor, an oncogene product such as v-Erb B2, an adhesion molecule, streptavidin (or a portion thereof) or an antibody.

The inventors have choosen preferably the F protein of Newcastle Disease Virus as viral target structure since it is considered to be advantageous to have more than one target structure in a viral molecule against which bispecific constructs can be made.

The F protein of the NDV virus was found to be particularly favourable as first bonding component. In a preferred embodiment, the single chain Fᵥ fragment directed against the F protein comprises the peptide sequence of figure 7b. This sequence was derived from a DNA library of the spleen of a mouse immunized with NDV. It was identified by recombinant phage cloning.

Alternatively, the first bonding component may be a single chain Fv fragment comprising the peptide sequence of Fig. 8b. This sequence was derived from the hybridoma HN.B which was obtained from an original hybridoma HN 1.4c as described in Iorio et al., J. Gen. Virol., 1986, pg. 1393. Of hundreds of clones tested by the inventors, the clon H9 represented by the sequence shown in Fig. 8b proofed to be most suitable.

It is therefore particularly preferred that the first bonding component is a single chain Fv fragment directed against the F protein of Newcastle Disease Virus on the cell surface of NDV infected cells or the first bonding component is a single chain Fv fragment comprising the peptide sequence of Fig. 8b. The F protein and the HN protein of NDV are particularly suitable for tumor targeting, as NDV infects only tumor cells.

The expression "second bonding component" in this aspect of the invention comprises any compound which may bind to a first bonding component, on the one hand, and to a costimulatory acting molecule of an effector cell, on the other hand. The linkage may be direct or indirect. The compound is perferably an antibody or a portion thereof which has a bonding domain. Such a portion is favourably a Fab', (Fab')₂, or (Fᵥ)₂ fragment. The Fᵥ fragment of an anti-CD19, anti-CD20, anti-CD22 antibody, and anti-CD28 antibody, respectively, has proved to be especially favorable. The antibodies or antibody fragments of the invention can be monovalent or multivalent. Multivalent antibodies or antibody fragments of the instant invention can be e.g. divalent, trivalent or tetravalent. One of skill in the art will appreciate that, as general rule, an antibody or antibody fragment of higher valency will bind to an antigen with greater avidity than an antibody or antibody fragment of lower valency will bind to the antigen.

It is particularly preferred that the second bonding component is a single chain Fᵥ fragment directed against the costimulatory acting molecule CD28 of effector cells and comprises the peptide sequence of Fig. 9b or of Fig. 10b. These sequence have proven to be particularly beneficial and give good results.

In another preferred embodiment the second bonding component is a ligand of a costimulatory acting molecule of an effector cell.

In an especially preferred embodiment, the compound is a protein or a fragment of a protein that is a ligand of a costimulatory acting molecule of an effector cell. Examples of such proteins include the B7.1 and B7.2. proteins, CD40 ligand and CD137 ligand. It has proved to be especially favorable when such a protein does not comprise a membrane-bound domain. In another escpecially preferred embodiment, the compound is a lymphokine, interferon or interleukin, e.g. GM-CSF or IL-2.

The invention is further directed to a bonding reagent having a first bonding component for a cell surface protein and a second bonding component for a costimulatory acting molecule of an effector cell, characterised in that the second bonding component is a single chain Fv fragment directed against the costimulatory acting molecule CD28 of effector cells and comprises the peptide sequence of Fig. 9b or of Fig. 10b.
These two particular sequences were found to be particularly useful for the present invention.

The expression "effector cell" comprises any cell taking part in an immune reaction. It is preferably a T cell.

The expression "costimulatory acting molecule" comprises any molecule of an effector cell, which by bonding or in another way can be induced to stimulate the effector cell. Such a molecule may be a receptor, for example. In the case of a T cell, particularly the receptors CD2-, CD3-, CD19-, CD20-, CD22-, CD26-, CD28-, and CTLA-4 as well as HSA (heat stable antigen) offer themselves as being favorable.

In the above bonding reagents according to the invention, the two bonding components may be connected directly or indirectly. In the latter case, this may be effected via a complex, consisting e.g. of streptavidin (avidin) as well as biotin and S protein as well as S peptide of the pancreatic ribonuclease, respectively. For this purpose, one of the two bonding components includes streptavidin (avidin) and S protein or a portion thereof, respectively, and the other bonding component includes biotin and S peptide or a portion thereof, respectively. A person skilled in the art is familiar with processes serving for linking the constituents of the individual complexes to the bonding components and reacting them with one another so as to form the complexes.

In a particularly preferred embodiment, the bonding components are part of a single, continuous polypeptide. Such bonding reagents are especially suitable for coupling to a tumor vaccine inn order to increase their efficiency. They may be employed as booster molecules either alone or in any combination. In a more particularly preferred embodiment, the polypeptide additionally comprises a linker. In a most preferred embodiment, the polypeptide comprises a plurality of antibody fragments and a linker arrayed linearly on the polypeptide from the amino terminus to the carboxy terminus in the following order: a V_{H} fragment of the second bonding component, a V_{L} fragment of the first bonding component, a linker, a V_{H} fragment of the first bonding component and a V_{L} fragment of the second bonding component.

Where the bonding components are indirectly bound to one another, bonding reagents are preferred in which one or both bonding components contain groups which are suitable to develop intermolecular disulfide bridges, i.e. disulfide bridges between bonding components of differing bonding reagents. Thus, e.g. bonding reagents which bond different, costimulatory acting molecules can be bonding while linked to a single cell surface protein. In many cases, this proves to be especially favourable to increase the immunogenicity of cells.

The bonding reagents of the invention optionally comprise other components as well. For example, the bonding reagents may be linked to a toxin, or one or more additional bonding components, or be radioactively labelled.

In a preferred embodiment, the first bonding component comprises an anti-NDV F protein antibody fragment and a cytokine-derived sequence, i.e. a sequence derived from GM-CSF or IL-2. In another preferred embodiment, the bonding agent comprises an anti-NDV F protein antibody fragment and another substance, e.g. a toxin or a radioactive substance.

The above bonding reagents may be produced according to conventional processes. For bonding reagents comprising two polypeptide chains, a process comprising the following steps is favorable:
(a) Insertion of a DNA encoding a first bonding component into a first expression vector, expression of the DNA, isolation of the expression product and purification thereof,
(b) Insertion of a DNA encoding a second bonding component into a second expression vector, expression of the DNA, isolation of the expression product and purification thereof, and
(c) linkage of the expression products of a) with that of b) as usual.

In steps (a) and (b), a first bonding component and a second bonding component, respectively, are produced by conventional DNA recombination techniques. The person skilled in the art is familiar with systems which can be used for the expression of the individual bonding components. Such a person knows vectors, expression control elements and cells which can be used for this purpose. It has proved to be favourable to provide both bonding components as Fᵥ fragments of antibodies.

The two Fᵥ fragment bonding components of the bonding reagent can also be combined in a single peptide chain. While it has been proposed to construct such a bonding reagent having a VH and a V_{L} chain of the different components (with the other V_{L} and VH chain being complemented by further plasmids), it was now found out that it will also be possible to link two complete Fᵥ fragments both consisting of a V_{H} and a V_{L} domain.

Where the bonding reagent comprises more than two polypeptide chains, the reagent can be prepared essentially as described above, wherein each polypeptide chain is expressed using an expression vector, then isolated and linked with the other polypeptide chains of the reagent.

Where the bonding reagent comprises a single polypeptide chain, it can be made using any technique known in the art. For example, it can be made recombinantly or synthetically. The reagent can be made recombinantly by, for example, inserting a DNA encoding a first bonding component and a second bonding component in an expression vector, expressing the DNA in vitro or in a suitable host cell and isolating the expression product.

Hence, in a further aspect, the invention is directed to a bonding reagent for costimulating an effector cell, which is characterised in that the bonding reagent is a polypeptide comprising in a first portion thereof a peptide sequence for a single chain Fᵥ fragment directed against a cell surface protein and comprising in a second portion thereof a peptide sequence for a single chain Fv fragment directed against a costimulatory acting molecule of the effector cell.

The expressions "cell surface protein" and "costimulatory acting molecule" have the same meaning as above identified with respect to the former aspects of the invention.

Preferably, the first portion is a single chain Fv fragment directed against the F protein or against the HN protein of Newcastle Disease Virus on the cell surface of NDV infected cells.

It is further preferred that the first bonding component is a single chain Fv fragment comprising the peptide sequence of Fig. 7b, in case it is directed against the F protein, or Fig. 8b, in case it is directed against the HN protein.

Further, it is also preferred that the second portion is a single chain Fv fragment directed against the CD3 cell surface protein or the CD28 cell surface protein of the effector cell, or is the GM-CSF protein or the IL-2 protein.

In particular, the second bonding component may be a single chain Fv fragment directed against the costimulatory acting molecule CD28 of effector cells and comprises the peptide sequence of Fig. 7b or of Fig. 8b.

Between the first and the second portions of the bonding reagent, there may be provided a spacer sequence. This spacer allows for a correct folding of both compounds without a high risk of interference. Also between the individual domains of every Fv fragments, there may be provided linker sequences.

The above bonding reagents may be produced according to conventional processes. A process comprising the following steps is favorable:
(a) Insertion of a DNA encoding the first bonding component and the second bonding component in an expression vector,
(b) expression of the DNA, and
(c) isolation of the expression product and purification thereof.

As in the above described process for manufacturing composite bonding reagents, the skilled person is familiar with appropriate methods for producing the bonding reagent according to this particular aspect of the invention.

The invention is further directed to expression plasmids for the expression of the above disclosed bonding reagents and components. According to the invention, an expression plasmid is provided which is characterised in that it contains a DNA encoding a first bonding component according to the invention in an expression unit and comprising the nucleotide sequence of Fig. 7a or of Fig. 8a.

Further, an expression plasmid is provided which is characterised in that it contains a DNA encoding a second bonding component according to the invention in an expression unit and comprising the nucleotide sequence of Fig. 9a or Fig. 10a.

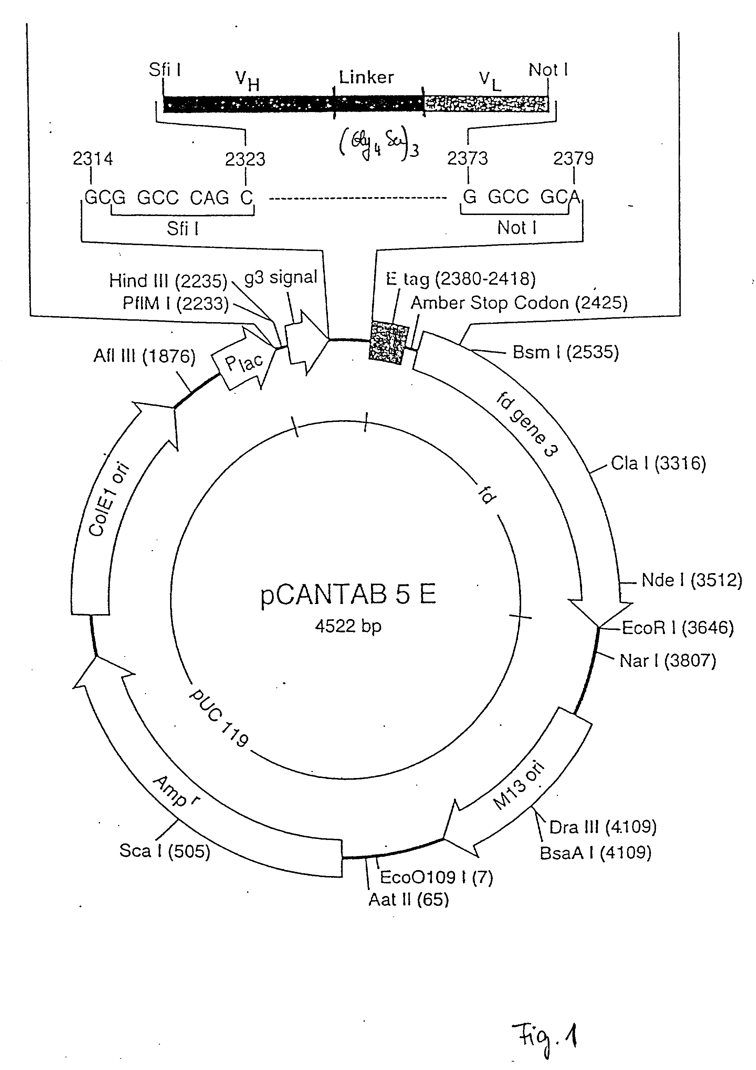

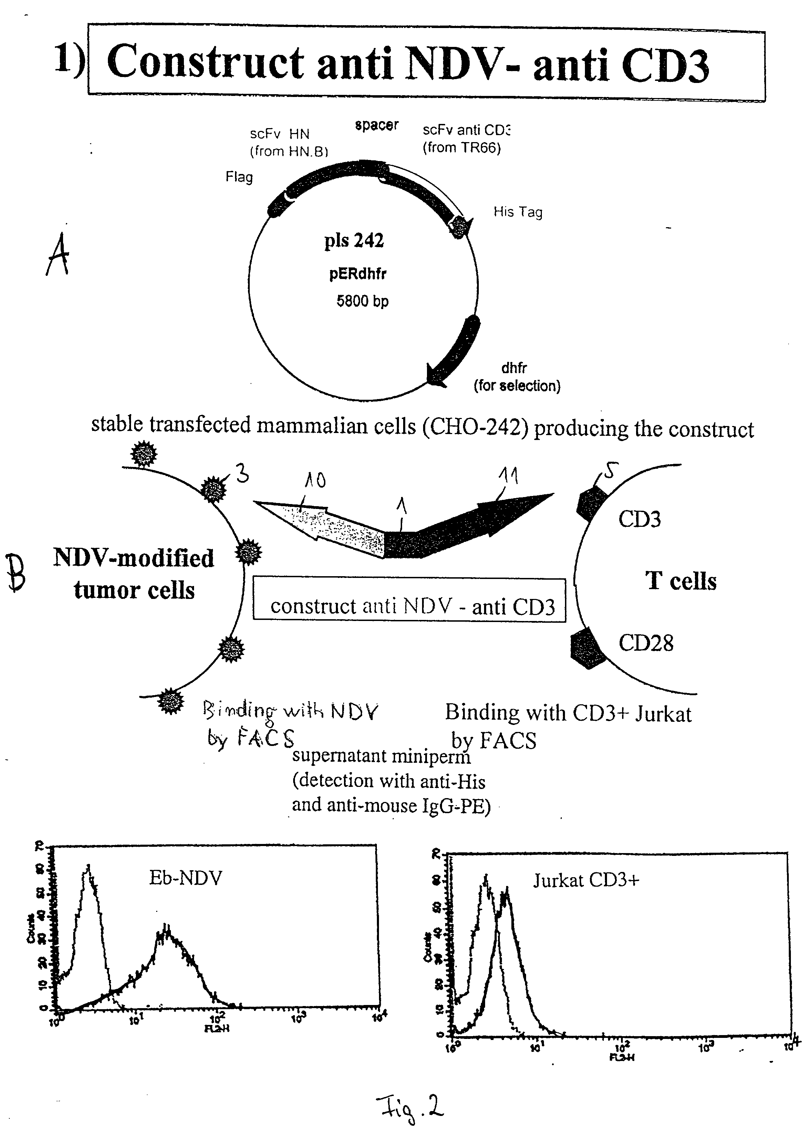

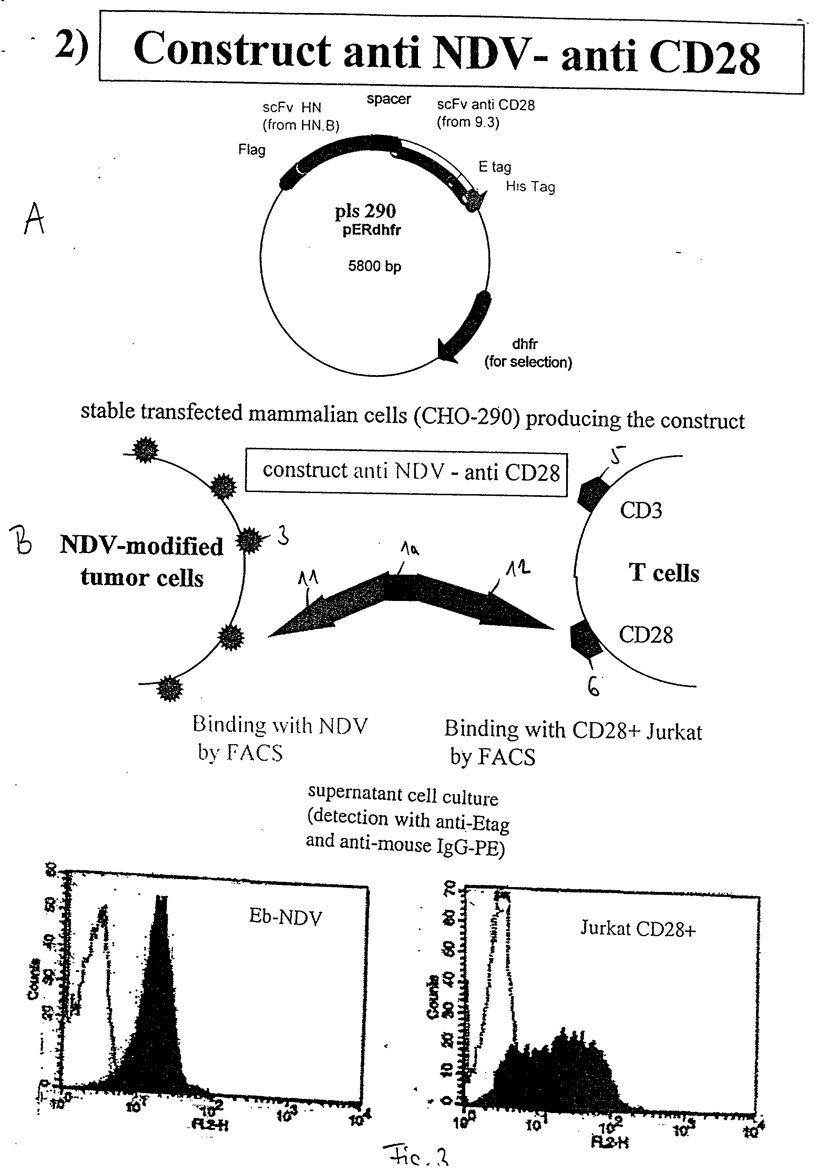

The expression plasmid according to the invention may have the structure shown in Fig. 1. This plasmid, called pCANTAB, has been particularly developped for effecting the invention and will be described herein by way of example.

Still further, there is provided according to the invention, an expression plasmid which is characterised in that it contains a DNA encoding a bonding reagent containing both bonding components in a single peptide, in an expression unit.

In a preferred embodiment, this expression plasmid according to the invention contains a DNA encoding as a first bonding component a single chain Fᵥ fragment directed against the F protein of Newcastle Disease Virus on the cell surface of NDV infected cells.

In particular, it may contain the nucleotide sequence of Fig. 7a encoding the first bonding component, and will hence comprise a sequence for an Fᵥ fragment directed against the F protein of NDV.

Alternatively, it may be preferred that this expression plasmid contains the nucleotide sequence of Fig. 8a for encoding the first bonding component, thus comprising a sequence for an Fᵥ fragment directed against the HN protein of NDV.

The expression plasmid according to this aspect of the invention may further contain the nucleotide sequence of Fig. 9a or Fig. 10a for encoding the second bonding component. Hence, in particularly preferred embodiments, an expression plasmid will be provided having a first sequence portion encoding either the particular Fᵥ fragment directed against the F protein or the HN protein Fᵥ from NDV, and a second sequence portion encoding an Fᵥ fragment directed against CD28 (in one of two preferred sequence variants) on an effector cell.

In a further aspect of the invention, the bonding reagent has been improved for an even better bonding to the targeted cells and/or the effector cells by providing a bonding reagent for costimulating an effector cell which has a first bonding component for a cell surface protein and a second bonding component for a costimulatory acting molecule of an effector cell, and which is characterised in that the first bonding component and/or the second bonding components have multivalent binding capabilities.

A conventional bonding reagent has bonding components which are able of binding to one target protein molecule at a time. This functionality is known as monovalent bonding. In order to improve the targetting process, it has been found out that it would be desirable to improve the crosslinking of the targeted and the effector cells. In order to achieve this goal, the inventors developped a concept of multivalent bonding components which are able of binding more than one of the respective molecules, which behaviour is called multivalent. Thereby, the avidity for bonding to e.g. virus infected tumor cells could be increased. Multivalent bonding components can have two, three, four or even more sites for bonding target molecules, named di-, tri-, tetravalent etc. According to the invention, the first bonding component, the second bonding component, or both components may be multivalent.

The bonding reagent according to this aspect of the invention may further be characterised in that either of the first bonding component and the second bonding component can comprise antibodies or portions thereof which have a binding domain. These antibodies can be aligned in the DNA in a head to tail or any other suitable arrangement, and can even be interleaved if this would improve the bonding capabilities of the inventive bonding reagent.

The bonding reagent may also be characterised in that the cell surface protein is a virus protein, a growth factor receptor, an oncogene product, an adhesion molecule, streptavidin or an antibody.

The costimulatory acting molecule may in turn be a CD2, CD3, CD19, CD22, CD26, CD28, or CTLA-4 receptor, while it is particularly preferred that the second bonding component is at least one single chain Fv fragment directed against the costimulatory acting molecule CD28 of effector cells and comprises the peptide sequence of Fig. 9b or of Fig. 10b.

In this aspect of the invention, the bonding components are preferably linked with one another via a complex.

The complex may for example consist of streptavidin (avidin) and biotin. It may also consist of S protein and S peptide, as was described in more detail above with reference to the inventive monovalent bonding reagents.

It is particularly preferred to combine the multivalency of the bonding reagent with the embodiments of the bonding reagent consisting of a single peptide chain combining all components and comprising a plurality of first portions with first bonding components and/or of second portions with second bonding components.

According to the invention, a vaccine having inactivated cells is provided, wherein one or more bonding reagent according to the invention are bound to a cell surface protein.

Such a vaccine preferably includes tumor cells which may originate from tumors removed by surgery or from an established cell line.

The (tumor) cells may be virus-modified. Lysates of (tumor) cells may also be present. NDV is used advantageously. It has been proved to be favourable for the bonding reagent or bonding reagents to be directed against a virus protein of NDV, particularly the F and HN proteins.

By means of the bonding reagents according to the invention, which are to be attached to (tumor) cells, it is possible to transmit signals to costimulatory acting molecules, e.g. receptors, of effector cells. Thus, effector cells do not only receive the signal transmitted by an antigen of (tumor) cells but are also costimulated. Therefore, the bonding reagents according to the invention are perfectly suitable to increase the immunogenicity of cells, particularly tumor cells. They represent a great improvement in the active immunization.

The invention is further directed to a use of a bonding reagent having a first bonding component for a cell surface protein and a second bonding component for a costimulatory acting molecule of an effector cell, for the production of a pharmaceutical composition for the treatment of tumor patients showing at least residual tumor cells.

The bonding reagent employed for the inventive use is preferably according to the above aspects of the invention, but may also be embodied using known bonding reagents. The application of such bonding reagents for treating tumor patients is a valid option which is independent from a tumor vaccine.

Hence, in a preferred embodiment, the pharmaceutical composition comprises a tumor specifc virus, and the bonding reagent has a first bonding component with a bonding specifity for proteins of said tumor specific virus which are located on the cell surface of infected cells.

As mentioned above, the pharmaceutical composition comprising a bonding reagent is suitable for treating tumors in a subject. In one embodiment, a bonding reagent or pharmaceutical composition comprising a bonding reagent is administered to a subject in need of such treatment. In a preferred embodiment, the subject is first treated with a tumor-specific NDV virus under conditions that allow the cells of the tumor to be infected by the virus and express virally encoded proteins. A reagent of the invention comprising a binding domain specific for an NDV protein expressed on the surface of the tumor cells, or a composition comprising a reagent, is then administered to the subject. This method can be combined with other methods of treating tumors, e.g. surgery, chemotherapy, radiation therapy and any other tumor treatment known in the art. It is frequently observed that surgery, chemotherapy etc. fail to completely remove the tumor. Therefore, the remaining tumor load (e.g. minimal residual disease ("M.R.D.") or metastases may be treated by administering to the subject the reagents or compositions described herein. In a preferred embodiment, the tumor is removed from the subject by surgery, a tumor-specific NDV virus is administered to the subject, and a reagent comprising a first bonding component for an NDV protein, or a composition comprising such a reagent, is administered to the subject.

The invention is further described with respect to the figures, which show:
Figure 1 shows plasmid pCANTAB 5E which is used for the construction and expression of several of the bonding cmoponents according to the invention;
Figure 2 depicts in part A) the layout of plasmid pERdhfr used for expressing a bifunctional bonding reagent with anti-HN and anti-CD3 Fᵥ fragments, in part B) a model for the functionality of this bonding reagent, and in part C) results for FACS binding assays;
Figure 3 depicts in part A) the layout of plasmid pERdhfr used for expressing a bifunctional bonding reagent with anti-HN and anti-CD28 Fᵥ fragments, in part B) a model for the functionality of this bonding reagent, and in part C) results for FACS binding assays;
Figure 4 depicts in part A) the layout of plasmid pERdhfr used for expressing a bifunctional bonding reagent with anti-F Fᵥ fragment and GM-CSF, in part B) a model for the functionality of this bonding reagent, and in part C) results for FACS binding assays and other binding results;
Figure 5 depicts in part A) the layout of plasmid pERdhfr used for expressing a bifunctional bonding reagent with anti-F Fᵥ fragment and IL-2, in part B) a model for the functionality of this bonding reagent, and in part C) results for FACS binding assays and other binding results;
Figure 6 schematically explains the overall concept of the present invention;
Figure 7 shows in part (a) the nucleotide sequence of scFᵥ NDV(F) (cl.14) derived from an NDV-immunized mouse and in part (b) the amino acid sequence of scFᵥ NDV (F);
Figure 8 shows in part (a) the nucleotide sequence of scFᵥ HN.B (cl. 9) derived from the hybridoma NH.B and in part (b) the amino acid sequence of scFᵥ HN.B;
Figure 9 shows in part (a) the nucleotide sequence of scFᵥ 28.6 (cl. C2) derived from the hybridoma 28.6 and in part (b) the amino acid sequence of scFv 28.6; and
Figure 10 depicts in part (a) the nucleotide sequence of scFᵥ 9.3 (cl. F11) derived from the hybridoma 9.3 and in part (b) the amino acid sequence of scFᵥ 9.3.

The below examples explain the invention in more detail. The following preparations and examples are given to enable those skilled in the art to more clearly understand and to practise the present invention. The present invention, however, is not limited in scope by the exemplified embodiments which are intended as illustrations of single asepcts of the invention only, and methods which are functionally equivalent are within the scope of the invention. Indeed, various modifications of the invention in addition to those described herein will become apparent to those skilled in the art from the foregoing description and accompanying drawings. Such modifications are intended to fall within the scope of the appended claims.

### Example 1:

### Construction of the template expression plasmid pCANTAB 5E

Figure 1 shows the the general layout of plasmid pCANTAB 5E which was used as a template plasmid for insertion of DNA sequences as depicted in Figs. 7, 8, 9 and 10. pCANTAB 5E is part of a recombinant phage antibody system commrecially available from Pharmacia under catalog No. 27-9401. The gene accession number is U14321. The construction of this plasmid has been described by McCafferty et al. in Apple.Biochem. Biotechnol., 47 (1994), pg. 157. Beyond the Hind II an d EcoR1 sites, the pCANTB 5E vector is the same as pUC119 except for the three ApaLI sites of pUC119 which have been deleted, and an XhoI site which has been added.

In Figure 1, the following abbreviations are used: Ampr: gene encoding ampicillin resistence; bp: base pairs; M13 ori: origin of DNA replication derived from phage M13; ColE1: origin of DNA replication for ColE1; E tag: petide tag sequence; g3 signal: signal sequence which drives in general the gene 3 protein gp3 to the periplasm; and fd gene 3: a gene coding maily for the minor capsid protein gp3 of phage f1, the sequence of which is very similar to the homologous protein of phage M13 and which could be complemented by the gene coming from the helper phage M13K07. It allows the expression of the insert scFᵥ at the surface of the phage. Recognition sites for restriction endonucleases are designated as usual and known to the skilled person.

Between the g3 signal and the E tag, there were inserted a sequence containing a Sfi I site and a further sequence containing a Not I site. Between these sites, Fᵥ fragments according to the invention were inserted through the use of the two restriction sites.

Particularly preferred was the inclusion of the DNA sequences shown in Figs. 7, 8, 9 and 10. These sequences can express VH and V_{L} domains of Fᵥ fragments which are connected through a linker having the amino acid sequence (Gly4Ser)3.

Into this plasmid were inserted scFᵥ fragments.

Plasmid pCANTAB, digested with SfiI and NotI, linearized and dephosphorylated, was purchased from Pharmacia.

The 750bp fragment encoding V_{H}, a (Gly4Ser)3 linker region and V_{L}, was isolated from the PCR products made using degenerated primers purchased from Pharmacia and cDNA obtained from hybridoma cells or the spleen cells of NDV immunized mice. This fragment was first digested with SflI (20 units) for 4 hours at 50 degrees Celsius with buffer 1 as provided by Pharmacia in a total volume of 85 microlitres. After this step, Buffer 2 (Pharmacia) and NaCl solution and 40 units of NotI digestion with NotI were added to the digestion reaction. After gel quantification of the digested PCR products, 150 ng of this fragments was ligated with 250 ng of the linear pCANTAB fragment using 6 units of T4 ligase (Biolabs) in the buffer supplied by the manufacturer in a total volume of 50 ul at 16 degrees Celsius during 1 hour. 50 ul of a 10(8)/ml preparation of electrocompetent E. Coli strain TG1 were electrotransformed with the ligation mixture (Dower et al., 1988, Nucleic Acid Research, 16: 6127-45, "High efficiency of transformation of E. Coli by high voltage electroporation"). Transformed cells were plated on SOBAG plate (containing 100 ug/ml amplicillin and 111,2 mM glucose). The selection of scFv binder candidate was made using the phage display system (Pharmacia cat nr 27-9401) as described in the instructions of this product and in the article of McCafferty et al. in (1994) Appl. Biochem. Biotechnol., 47: 157-173.

### Example 2:

### Construction of an expression plasmid (pls242) expressing a bifunctional antibody with anti NDV and anti CD3, as bonding reagent

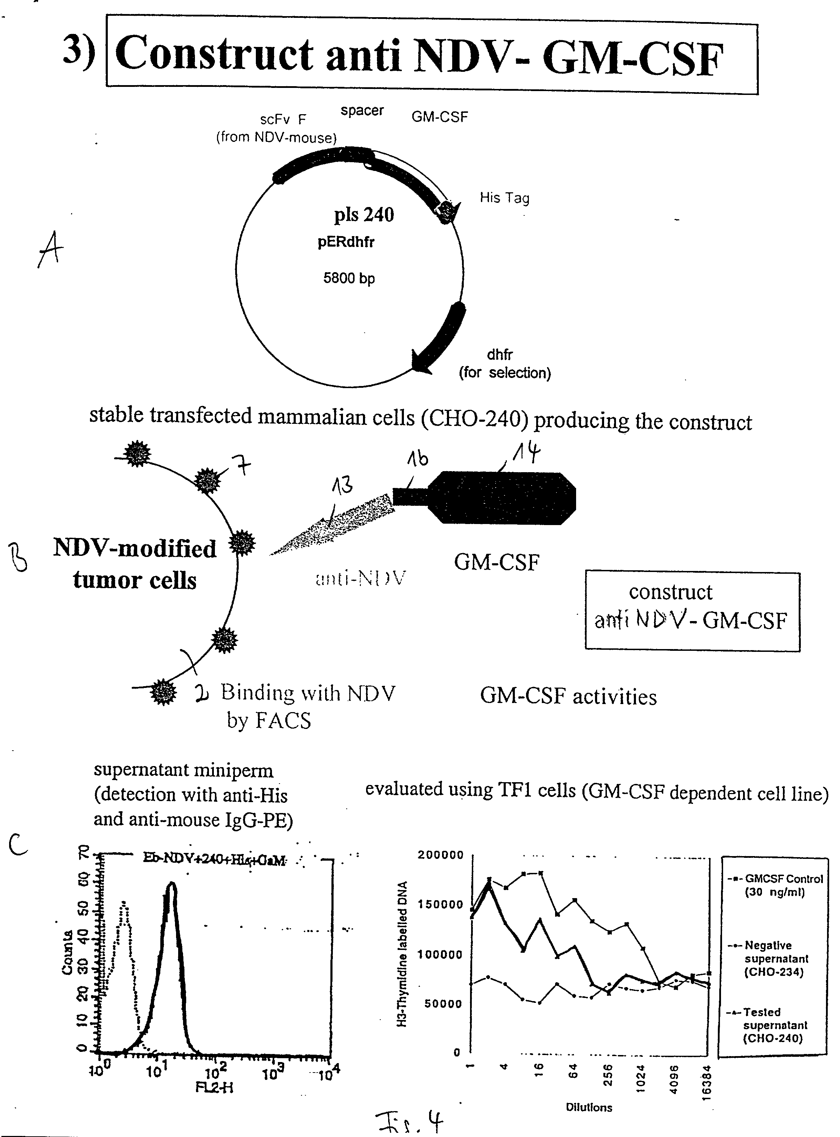

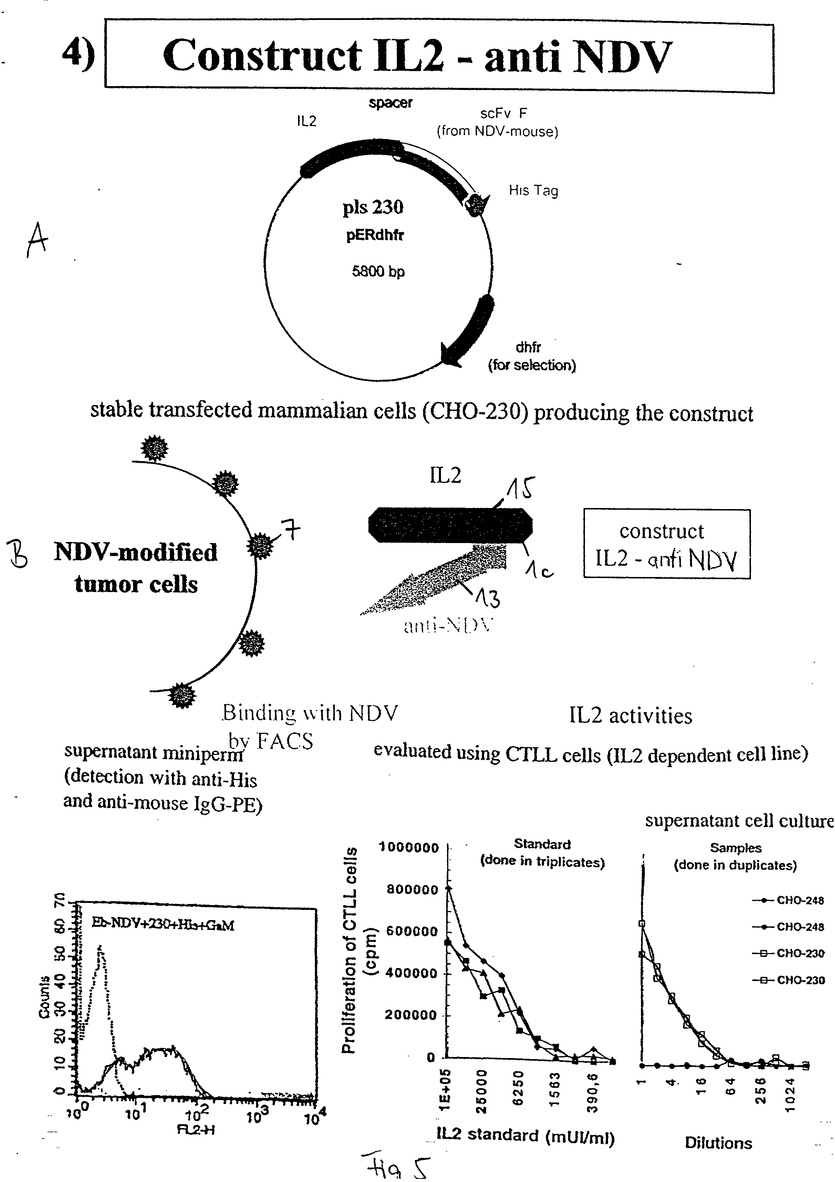

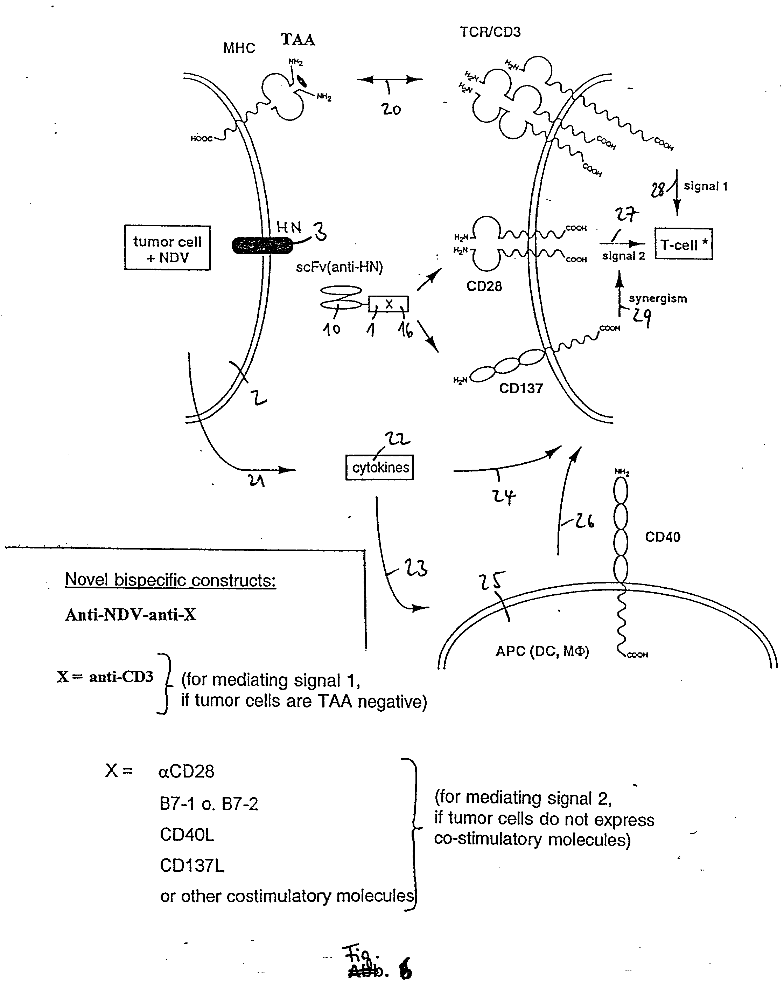

As shown in Fig. 2A, into plasmid pERdhfr, there was inserted a construct of a sequence coding for a single chain Fv fragment directed against the HN protein of NDV, and a sequence coding for a single chain Fᵥ fragment directed against the CD3 protein of effector cells.

The vector used for the construction ist commercially avaliable, e.g. from ATCC as pSV2-dhfr5 (ATCC 37146) The construction of this vector has been described by Mack et al., PNAS USA, 92 (1995), pg. 7021.

The plasmid 242 has been obtained using molecular biological techniques (Molecular Cloning: a laboratory manual, Sambrook, Fritsch, Maniatis) by inserting in the pERdhfr vector from the Nterminus to the C terminus:
- A mammalian signal sequence leading to the secretion of the desired protein sequence as described in von Heijne (1986) "A new method for predicting signal sequence cleavage sites" Nucleic Acids Research vol14 nr 11p. 4683-4690,
- A fragment corresponding to the Flag sequence allowing the detection of the protein by using an antibody directed against this sequence,
- An scFv directed towards the HN protein of NDV (as shown in Fig. 8A),
- A spacer made by Gly and Ser residues,
- An scFv directed towards the human CD3, made from the TR66 hybridoma, as described in Lanzavecchia and Scheidegger, 1987, (1987), Eur. J. Immunol., 17: 105-111. "The use of hybrid hybridomas to target human cytotoxic T lymphjocytes.", realisation of the scFv from the hybridoma described in Traunecker et al., (1991) The EMBO Journal, vol 10, no. 12, p 3677-9 "Bispecific single chain molecules (Janusins) target cytotoxic lymphocytes on HIV infected cells"),
- An polyhistidine tail made of 6 consecutive histidine amino acids allowing the purification of the desired protein using a Cu column.

The plasmid further contains a dhfr gene for selection of positive clones.

The expression plasmid is stably transfected into mammalian cells by the following steps and produces the bifunctional construct.
- Dhfr deficient chinese hamster cells CHO (ATCC CRL-9096) or DSMZ (ACC 126) are electrotransfected (Jorgensen et al., Cell 48: 185- (1987); Tykocinski et al., PNAS USA, 85, 3555- (1988); Zerbib et al., Biochem. Biophys. Res. Comm., 129:611- (1985) with the above described plasmid pls 242. Positive clones are selected in medium containing glutamine, Hepes, dialysed fetal calf serum (FCS without desoxyribonucleotide and ribonucleotide) and methotrexate (which lead to an increase of copy of plasmid per cells and then to an higher level of production when the concentration of methotrexate is increasing). After selection of clones showing a good level of production of the expected product, the cells are cultured in a high density bioreactor (Miniperm, Haereus or Integra flask, Integra Biosciences).
- Purification of the desired protein from the cell supernatant is made using a Cu column. After wash of the column, the elution is realised by washing the column with a imidazol solution following a stepwise increase of concentration of this reagent.

### Example 3:

### Bindung of the bonding reagent of Example 2 to cells

The principle by which this construct will act on cells, is shown in Fig. 2B. The bonding reagent 1 has two bonding components, one for the HN protein 10 and one for the CD3 protein 11. Bonding component 10 will bind to HN protein molecules 3 present on the cell surface of NDV modified tumor cells 2. Bonding component 11 will bind to CD3 molecules 5 on the cell surface of T cells. Other cell surface proteins like CD28 (6) will not be involved in this embodiment of the invention.

Fig. 2C shows results of FACS binding assays. To analyze the binding of the obtained protein to cells, the protein of the supernatant of culture or the purified protein is incubated for 20 minutes on ice
- with tumor cells which have been modified by infection with NDV or
- with Jurkat cells, a human T cell leukemia (Weiss A et al. "The role of T3 surface molecules in the activation of human T cells: a two-stimulus requirement for IL-2 production reflects events occurring at a pre-translational level. "J. Immunol. 133: 123-128, 1984) expressing the CD3 molecule at the cell surface.

The addition of a second antibody recognizing the tag sequence (either the Flag sequence located at the N terminus either an His sequence located at the C terminus) and a fluorescent-labelled antibody directed towards the second antibody allows the detection of the protein at the surface of the NDV-modified tumor cells or of the CD28 positive cells by flow cytometry. As negative controls cells are used which have not been modified by NDV and which do not express the CD3 molecule at their surface.

For the FACS, 200 microlitres of cell culture supernatant (unconcentrated) of the Miniperm reactor with 5 x 100000 cells are used. For the Eb-NDV FACS; Eb cells infected with live NDV following a standard protocoll are used, while for CD3, Jurkat CD3+ cells (which have been sorted for a high level of expression of CD3 molecules) are employed. In both cases, uninfected Eb cells are used as negative controls. After incubation, anti-His monoclonal antibodies from Dianova (1: 250, 50 microlitres) and goat anti-mouse-PE antibodies (1:100, 50 microlitres) are added.

The binding of the protein of the supernatant of the transfection obtained with the pls242 with Eb-NDV and with Jurkat-CD3+ cells is observed, but no binding is found for the noninfected Eb cells.

### Example 4:

### Construction of an expression plasmid (p1s290) expressing a bifunctional antibody with anti NDV and anti CD28, as bonding reagent

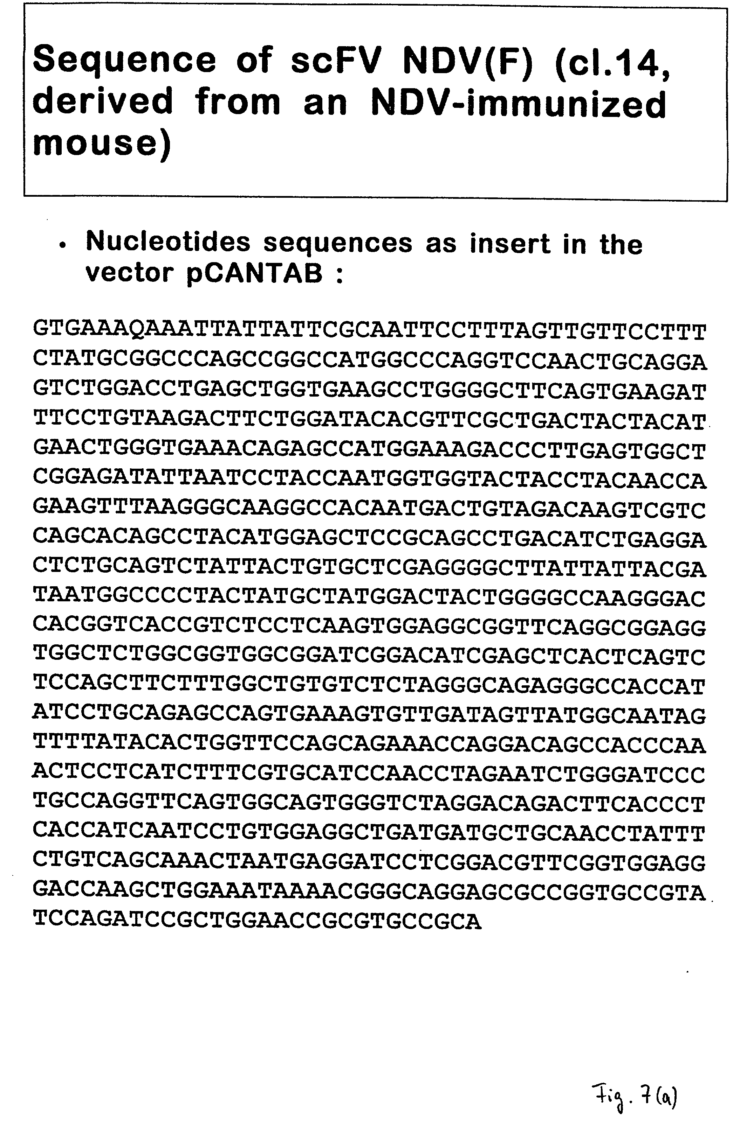

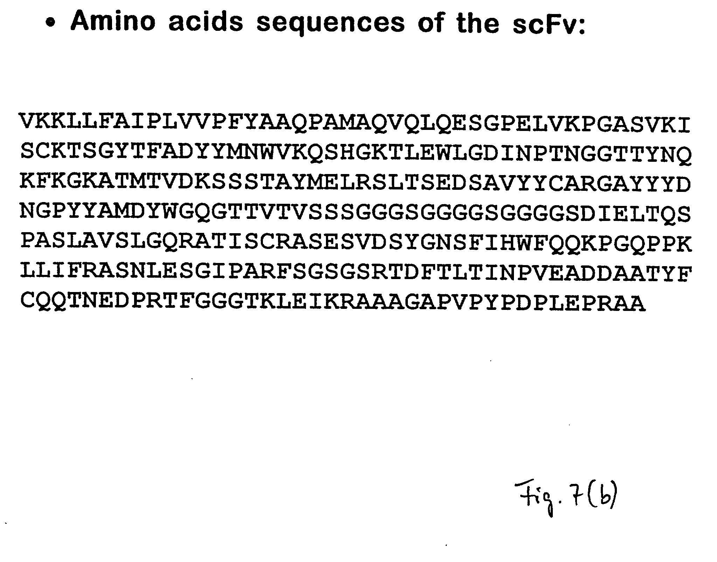

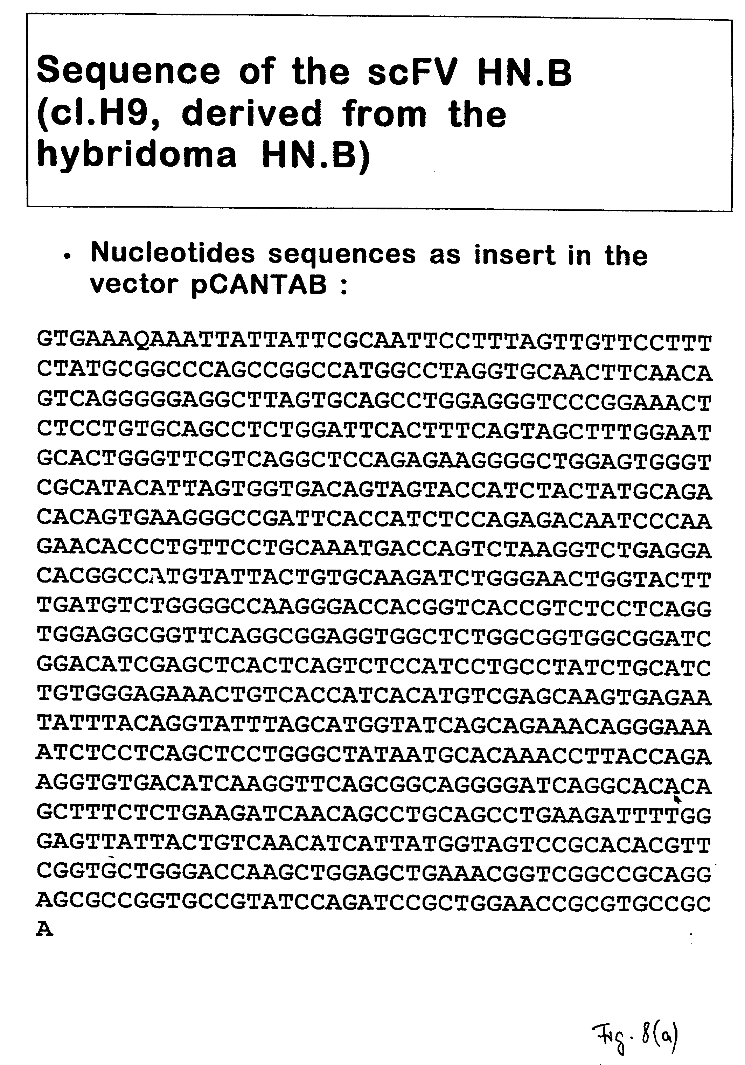

As shown in Fig. 3A, into plasmid pERdhfr, there was inserted a construct of a sequence coding for a single chain Fv fragment directed against the HN protein of NDV, and a sequence coding for a single chain Fv fragment directed against the CD28 protein of effector cells.

The plasmid 290 has been obtained using molecular biological techniques(Molecular Cloning: a laboratory manual, Sambrook, Fritsch, Maiatis) by inserting in the pERdhfr vector from the N terminus to the C terminus:
- A mammalian signal sequence leading to the secretion of the desired protein sequence as described in von Heijne (1986) "A new method for predicting signal sequence cleavage sites" Nucleic Acids Research vol14 nr. 11 p. 4683-4690.
- A fragment corresponding to the Flag sequence allowing the detection of the protein by using an antibody directed against this sequence,
- An scFv directed towards the HN protein of NDV (as shown in Fig. 8a
- A spacer made by Gly and Ser residues,
- The scFv 9.3 (Figure 10a, an antibody fragment that specifically recognized the human CD28 molecule at the surface of t cells. This scFv was obtained from the hybridoma 9.3 described by J.A. Hansen Immunogenetics, 10: 247-260 (1980) "Monoclonal antibodies identifying a novel T-cell antigen and Ia antigens of human lympohocytes",
- An amino acid sequence denominated Etag sequence towards which a commercial monoclonal antibody can be purchased from Pharmacia (cat 27-9412-01 or 27-9412-02),
- An polyhistidine tail made of 6 consecutive histidine amino acids allowing the purification of the desired protein using a Cu column.

The plasmid further contains a dhfr gene for selection of positive clones.

The expression plasmid is stably transfected into mammalian cells by the following steps and produces the bifunctional construct.
- Dhfr deficient chinese hamster cells CHO (ATCC CRL-9096) or DSMZ (ACC126) were electrotransfered (Jorgensen et al., Cell 48: 185- (1987); Tykocinski et al., PNAS USA, 85, 3555- (1988); Zerbib et al., Biochem. Biophys. Res. Comm., 129: 611- (1985) with the above described plasmid pls 290. Positive clones are selected in medium containing glutamine, Hepes, dialysed fetal calf serum (FCS without desoxyribonucleotide and ribonucleotide) and methotrexate (which lead to an increase of copy of plasmid per cells and then to an higher level of production when the concentration of methotrexate is increasing). After selection of clones showing a good level of production of the expected product, the cells are cultuired in a high density bioreactor (Minipem, Haeraeus or Integra flask, Integra Biosciences).
- Purification of the desired protein from the cell supernatant is made using a Cu column. After wash of the columm, the elution is realised by washing the column with an imidazol solution following a stepwise increase of concentration of this reagent.

### Example 5:

### Binding of the bonding reagent of Example 4 to cells

The principle by which this construct will act on cells, is shown in Fig. 3B. The bonding reagent 1a has two bonding components, one for the HN protein 10 and one for the CD28 protein 12. Bonding component 1a will bind to HN protein molecules 3 present on the cell surface of NDV modified tumor cells 2. Bonding component 12 will bind to CD28 molecules 5 on the cell surface of T cells. Other cell surface proteins like CD3 (6) will not be involved with this embodiment of the invention.

Fig. 3C shows results of FACS binding assay. To analyze the binding of the obtained protein to cells, the supernatant of culture or the purified protein is incubated for 20 minutes on ice
- with tumor cells which have been modified by infection with NDV, or
- with Jurkat cells, a T cell lymphoma (ATCC) expressing the CD28 molecule at the cell surface.
The addition of a second antibody recognizing the tag sequence (either the Flag sequence located at the N terminus either an His sequence located at the C terminus or the Etag sequence) and an fluorescent-labelled antibody directed towards the second antibody allows the detection of the protein at the surface of the NDV-modified tumor cells or of the CD28 positive cells by flow cytometry.
As negative controls, cells are used which have not be modified by NDV and which are not expressing the CD28 molecule at their surface.

For the FACS, 200 microlitres of cell culture supernatant (unconcentrated) of the Miniperm reactor with 5 x 100000 cells are used. For the Eb-NDV FACS; Eb cells infected with live NDV following a standard protocoll are used, while for CD28, Jurkat CD28+ cells (which have been sorted for a high level of expression of CD28 molecules) are employed. In both cases, uninfected Eb cells are used as negative controls. After incubation, anti-E-Tag monoclonal antibodies (1: 150, 50 microlitres) and goat anti-mouse-PE antibodies (1:100, 50 microlitres) are added.

The binding of the protein of the supernatant of the transfection obtained with the pls290 with Eb-NDV and with Jurkat-CD28+ cells is observed, but no binding is found for the noninfected Eb cells.

### Example 6:

### Construction of an expression plasmid (pls240) expressing a bonding reagent with anti NDV and GM-CSF

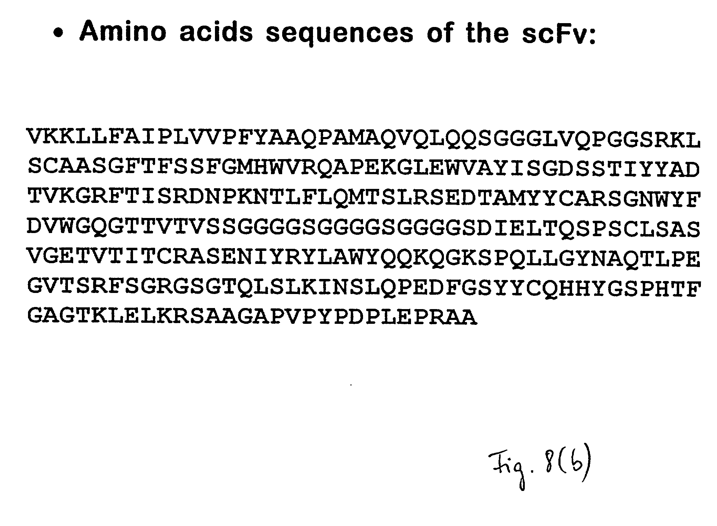

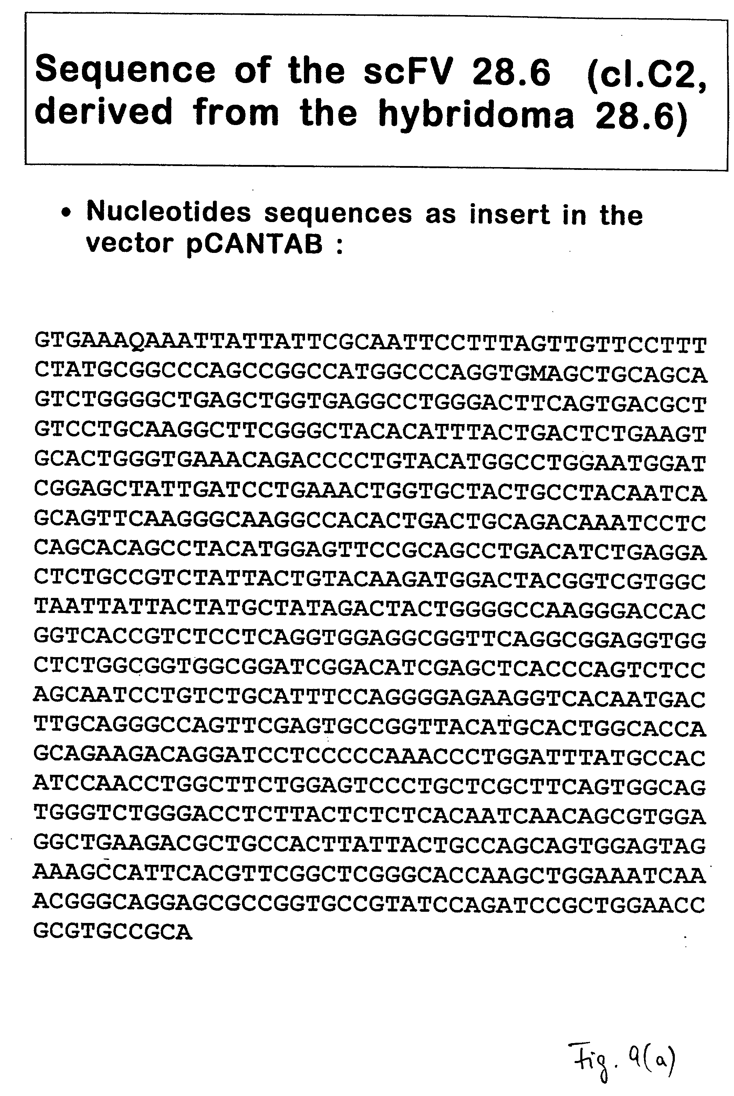

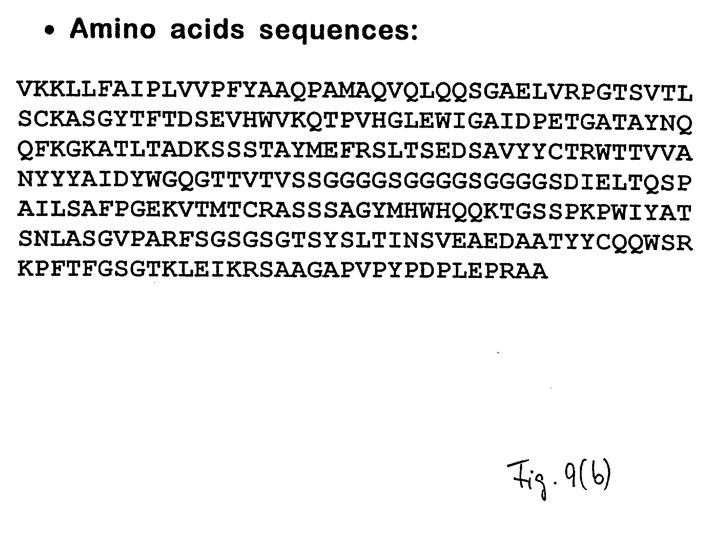

As shown in Fig. 4A, into plasmid pERdhfr, there was inserted a construct of a sequence coding for a single chain Fv fragment directed against the F protein of NDV, and a sequence coding for GM-CSF.

The plasmid 240 has been obtained using molecular biological techniques (Molecular Cloning: a laboratory manual, Sambrook, Fritsch, Maniatis)by inserting in the pERdhfr vector from the N terminus to the C terminus:
- A mammalian signal sequence leading to the secretion of the desired protein sequence as described in von Heijne (1986 "A new method for predicting signal sequence cleavage sites" Nucleic Acids Research vol 14 nr. 11 p. 4683-4690.
- An scFv directed towards the F protein of NDV (as shown in Fig. 7a)
- A spacer made by Gly and Ser residues
- The human cDNA of GM-CSF (Lee et al., PNAS USA, 82: 4360-4364 (1985); Wong et al., Science, vol: 228, p. 810-814 (1985)) GenBank Accession number : M 11220 obtained from the ATCC vector pcD-hGM/EO-CSF (ATCC No. 57594)
- An polyhistidine tail made of 6 consecutive histidine amino acids allowing the purification of the desired protein using a Cu column.

GM-CSF acts on target cells through specific surface membrane receptors. The cDNA for two human GM-CSF receptor components have been cloned (Gearing et al., EMBO J. 8 (1989), pg. 3667; and Hayashida et al., PNAS USA 87 (1990), pg. 9655). There is an alpha subunit that binds GM-CSF with low affinity and a beta chain that does not bind the ligand, but cooperates with the alpha chain to increase receptor affinity by reducing the rate of ligand dissociation from the complex. Several isoforms of the GM-CSF receptor alpha unit arising by alternative splicing have been identified.

The plasmid further contains a dhfr gene for selection of positive clones.

The expression plasmid is stably transfected into mammalian cells by the following steps and produces the bifunctional construct.
- Dhfr deficient chinese hamster cells CHO (ATCC CRL-9096) or DSMZ (ACC126) are electrotransfected (Jorgensen et al., Cell 48: 185- (1987; Tykocinski et al., PNAS USA, 85, 35555- (1988); Zerbib et al., Biochem. Biophys. Res. Comm., 129: 611- (1985) with the above described plasmid pls 240. Positive clones are selected inmedium containing glutamine, Hepes, dialysed fetal calf serum (FCS without desoxyribonucleotide and ribonucleotide) and methotrexate (which lead to an increase of copy of plasmid per cells and then to an higher level of production when the concentration of methotrexate is increasing). After selection of clones showing a good level of production of the expected product, the cells are cultured in a high density bioreactor (Minipem, Hareaus or Integra flask, Integra Biosciences).
- Purification of the desired protein from the cell supernatant is made using a Cu column. After wash of the column, the elution is realised by washing the column with an imidazol solution following a stepwise increase of concentration of this reagent.

### Example 7:

### Binding of the bonding reagent of Example 6 to cells

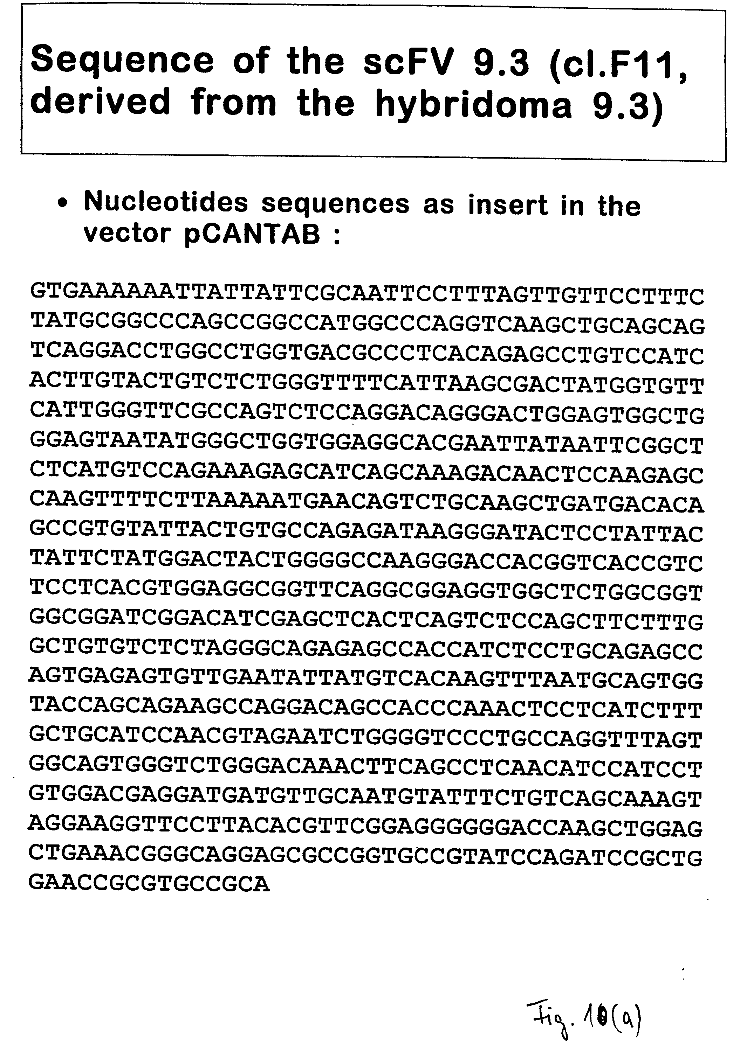

The principle by which this construct will act on cells, is shown in Fig. 4B. The bonding reagent 1b has a bonding component for the F protein 13 the bonding component GM-CSF. Bonding component 13 will bind to F protein molecules 7 present on the cell surface of NDV modified tumor cells 2. Bonding component 14 will bind to proper target cells.

Fig. 4C shows results of FACS binding assay (left side) and of GM-CSF activities (right side).

To analyze the binding of the obtained protein to NDV, the supernatant of the culture or the purified protein is incubated for 20 minutes on ice with tumor cells which have been modified by infection with NDV.

The addition of a second antibody recognizing the His sequence located at the C terminus and an fluorescent-labelled antibody directed towards the second antibody allows the detection of the protein at the surface of the NDV-modified tumor cells by flow cytometry.

As negative controls cells are used which have not be modified by NDV.

The functional activities of the obtained protein have been tested in proliferation assay using TF1 cells (ATCC#CRL-2003) which is a human cell line whose growth is dependent on the presence and on the concentration of GM-CSF in the culture medium and 3H thymidine as labeling reagent.

For the FACS, 200 microlitres of cell culture supernatant (unconcentrated) of the Miniperm reactor with 5 x 100000 cells are used. Eb cells infected with live NDV following a standard protocoll are used. Uninfected Eb cells are used as negative controls. After incubation, anti-His monoclonal antibodies (1: 250, 50 microlitres) and goat anti-mouse-PE antibodies (1:100, 50 microlitres) are added.

The binding of the protein of the supernatant of the transfection obtained with the pls240 with Eb-NDV cells is observed, but no binding is found for the noninfected Eb cells.

For the proliferation assay data, proliferation of TF-1 cells (which are GM-CSF dependent cells) is determined through tritium radiolabel thymidin uptake.

GM-CSF activity is detected in the supernatant of the transfection made with pls240. A GM-CSF standard is used as positive control and a supernatant of mock-transfected cells as negative controls.

### Example 8:

### Construction of an expression plasmid (pls230) expressing a bonding reagent with anti NDV and IL-2

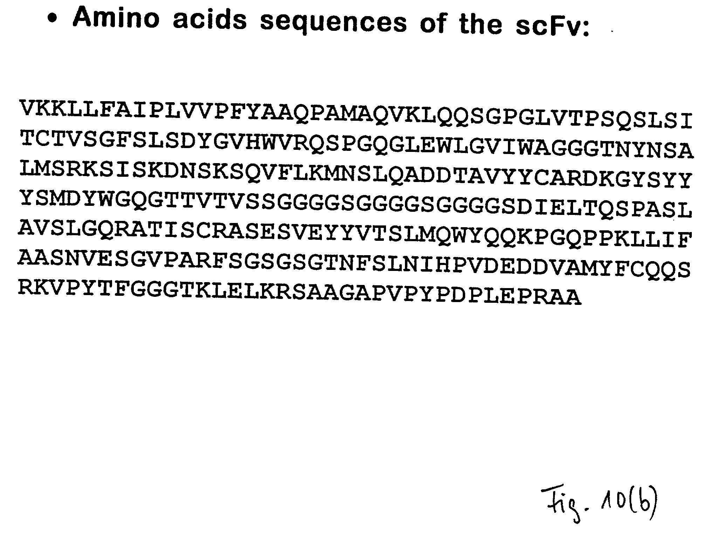

As shown in Fig. 5A, into plasmid pERdhfr, there was inserted a construct of a sequence coding for a single chain Fv fragment directed against the F protein of NDV, and a sequence coding for the IL-2 protein.

The pls 230 has been obtained using molecular biological techniques (Molecular Cloning: a laboratory manual, Sambrook, Fritsch, Maniatis) by inserting in the pERdhfr vector from the N terminus to the C terminus:
- A mammalian signal sequence leading to the secretion of the desired protein sequence as described in von Heijne (1986) "A new method for predicting signal sequence cleavage sites" Nucleic Acids Research vol. 14 nr. 11 p. 4683-4690.
- The human cDNA of IL2 (Holbrook et al., Nucleic acid research 12 (12), 5005-5013 (1984; Degrave et al., EMBP J. 2(12), 2349-2353 (1983); Taniguchi et al., Nature 302:5906 (1983)) GenBank Accesion number: X00695; X00200; X00201; X00202
- A spacer made by Gly and Ser residues
- The scFv directed towards the F protein of NDV (as shown in Fig. 7a)
- An polyhistidine tail made of 6 consecutive histidine amino acids allowing the purification of the desired protein using a Cu column.

The plasmid further contains a dhfr gene for selection of positive clones.

The expression plasmid is stably transfected into mammalian cells by the following steps and produces the bifunctional construct.
- Dhfr deficient chinese hamster cells CHO (ATCC CRL-9096 or DSMZ (ACC126) are electrotransfected (Jorgensen et al., Cell 48: 185- (1987); Tykocinski et al., PNAS USA, 85, 3555- (1988); Zerbib et al., Biochem. Biophys.Res. Comm., 129:611- (1985) with the above described plasmid pls 230. Positive clones were selected in medium containing glutamin, Hepes, dialysed fetal calf serum (FCS without desoxyribonucleotide and ribonucleotide) and methotrexate (which lead to an increase of copy of plasmid per cells and then to an higher level of production when the concentration of methotrexate is increasing). After selection of clones showing a good level of production of the expected product, the cells are cultured in high density bioreactor (Minipem, Hareaus or Integra flask, Integra Biosciences).
- Purification of the desired protein from the cell supernatant is made using a Cu column. After wash of the column, the elution is realised by washing the column with an imidazol solution following a stepwise increase of concentration of this reagent.

### Example 9:

### Binding of the bonding reagent of Example 8 to cells

The principle by which this construct will act on cells, is shown in Fig. 5B. The bonding reagent 1c has two bonding components for the F protein 13 and and IL-2 (15). Bonding component 13 will bind to F protein molecules 7 present on the cell surface of NDV modified tumor cells 2. Bonding component 15 will bind to compatible molecules on the cell surface of appropriate cells.

Fig. 5C shows results of a FACS binding assay.

To analyze the binding of the obtained protein to NDV, the protein of the supernatant of culture or the purified protein is incubated for 20 minuts on ice with tumor cells which have been modified by infection with NDV.
The addition of a second antibody recognizing the His sequence located at the C terminus and an fluorescent-labelled antibody directed towards the second antibody allows the detection of the protein at the surface of the NDV-modified tumor cells by flow cytometry.
As negative controls are used cells which have not be modified by NDV.

The functional activities of the obtained protein has been tested in proliferation assay using a murine lymphoblastic T cell line (CTLL) whose growth is dependent on the presence and on the concentration of IL2 in the culture medium. The growth of these cells is followed using 3H thymidine as labeling reagent.

For the FACS, 200 microlitres of cell culture supernatant (unconcentrated) of the Miniperm reactor with 5 x 100000 cells are used. Eb cells infected with live NDV following a standard protocoll are used. Uninfected Eb cells are used as negative controls. After incubation, anti-His monoclonal antibodies from Dianova (1: 250, 50 microlitres) and goat anti-mouse-PE antibodies (1:100, 50 microlitres) are added.

The binding of the protein of the supernatant of the transfection obtained with the pls230 with Eb-NDV cells is observed, but no binding is found for the noninfected Eb cells.

For the proliferation assay data, proliferation of CTLL cells (which are IL-2 dependent cells) is determined through tritium radiolable thymidin uptake.

IL-2 activity is detected in the supernatant of the transfection made with pls230. A supernatant of mock-transfected is used cells as negative controls.

Finally, Figure 6 presents an overview of the concepts of the present invention. While not wanting to be bound to any particular theory, a mechanism for the functioning of the present invention is introduced.

Figure 6 illustrates the interaction of the bonding reagent 1 according to the invention, with cell surface proteins 3 like the HN protein, on cells 2 on the one hand, and with costimulatory acting molecules like CD28 or CD137 on the surface of effector cells 4, on the other hand. The bonding reagent 1 comprises a first bonding component for bonding to protein 3, and e second bonding component 16 for bonding to a molecule on the effector cell. Bonding component 16 may be suitable for bonding the CD3 complex, e.g. an anti-CD3 Fv fragment as described above. This will activate a first signal 28 which in turn will activate the T cell 4.

Alternatively, as depicted in Figure 6, bonding component 16 may also be provided to bind to CD28, e.g. as a Fᵥ fragment directed against CD28 showing e.g. the peptide sequences presented in Figures 9b and 10b. The binding to CD28 will stimulate a second signal path 27 which will in turn stimulate the T cell.

Further, when using a CD137L protein as a bonding component 16, the bonding will stimulate the signal Path 29 which adds a synergistic effect to the stimulation of signal path 27.

The bonding component 16 may also be a B7-1, B7-2 or CD40L protein.

Further, through signal 21, the tumor cell may activate the corporal cytokine pool 22 which will through signal 23 stimulate further cells 25 like antigen present cells, e.g. DC or M-Phi. These may than stimulate through signal 26 the T cells 4. The T cells 4 may also be stimulated directly by cytokines through signal 24.

## Claims

1. A bonding reagent having a first bonding component for a cell surface protein and a second bonding component for a costimulatory acting molecule of an effector cell, **characterised in that** the first bonding component is a single chain Fᵥ fragment directed against the F protein of Newcastle Disease Virus on the cell surface of NDV infected cells or the first bonding component is a single chain Fᵥ fragment comprising the peptide sequence of Fig. 8b.

2. The bonding reagent according to claim 1, **characterised in that** the single chain Fᵥ fragment directed against the F protein comprises the peptide sequence of Fig. 7b.

3. The bonding reagent according to claim 1 or 2, **characterised in that** the costimulatory acting molecule is a CD2, CD3, CD19, CD20, CD22, CD26, CD28, or CTLA-4 receptor or HSA.

4. The bonding reagent according to claim 3, **characterised in that** the second bonding component is a single chain Fᵥ fragment directed against the costimulatory acting molecule CD28 of effector cells and comprises the peptide sequence of Fig. 9b or Fig. 10b.

5. The bonding reagent according to claim 1 or 2, **characterised in that** the second bonding component is a CD40L protein, a CD137L protein, or a B7 protein.

6. A bonding reagent having a first bonding component for a cell surface protein and a second bonding component for a costimulatory acting molecule of an effector cell, **characterised in that** the second bonding component is a single chain Fᵥ fragment directed against the costimulatory acting molecule CD28 of effector cells and comprises the peptide sequence of Fig. 9b or of Fig. 10b.

7. The bonding reagent according to claim 6, **characterised in that** the cell surface protein is a virus protein, a growth factor receptor, an oncogene product, an adhesion molecule, streptavidin or an antibody.

8. The bonding reagent according to claim 6 or 7, **characterised in that** the first bonding component is a single chain Fv fragment directed against the F protein of Newcastle Disease Virus on the cell surface of NDV infected cells or the first bonding component is a single chain Fᵥ fragment comprising the peptide sequence of Fig. 8b.

9. A bonding reagent for costimulating an effector cell, **characterised in that** the bonding reagent is a polypeptide comprising in a first portion thereof a peptide sequence for a single chain Fᵥ fragment directed against a cell surface protein and comprising in a second portion thereof a peptide sequence for a single chain Fᵥ fragment directed against a costimulatory acting molecule of the effector cell.

10. The bonding reagent according to claim 9, **characterised in that** the first portion is a single chain Fᵥ fragment directed against the F protein or against the HN protein of Newcastle Disease Virus on the cell surface of NDV infected cells.

11. The bonding reagent according to claim 10, **characterised in that** the first bonding component is a single chain Fᵥ fragment comprising the peptide sequence of Fig. 7b or Fig. 8b.

12. The bonding reagent according to any of claims 9 to 11, **characterised in that** the second portion is a single chain Fᵥ fragment directed against the CD3 cell surface protein or the CD28 cell surface protein of the effector cell, or is the GM-CSF protein or the IL-2 protein.

13. The bonding reagent according to claim 12, **characterised in that** the second bonding component is a single chain Fᵥ fragment directed against the costimulatory acting molecule CD28 of effector cells and comprises the peptide sequence of Fig. 9b or of Fig. 10b.

14. The bonding reagent according to any of claims 9 to 13, **characterised in that** between the first and the second portions of the bonding reagent, there is provided a spacer sequence.

15. An expression plasmid, **characterised in that** it contains a DNA encoding a first bonding component according to any of claims 1 to 5 in an expression unit and comprises the nucleotide sequence of Fig. 7a or Fig. 8a.

16. An expression plasmid, **characterised in that** it contains a DNA encoding a second bonding component according to any of claims 6 to 8 in an expression unit and comprises the nucleotide sequence of Fig. 9a or Fig. 10a.

17. The expression plasmid according to claim 15 or 16, **characterised in that** it has the structure shown in Fig. 1.

18. An expression plasmid, **characterised in that** it contains a DNA encoding a bonding reagent according to any of claims 9 to 14 in an expression unit.

19. The expression plasmid according to claim 18, **characterised in that** it contains a DNA encoding as a first bonding component a single chain Fᵥ fragment directed against the F protein of Newcastle Disease Virus on the cell surface of NDV infected cells.

20. The expression plasmid according to claim 19, **characterised in that** it contains the nucleotide sequence of Fig. 7a for encoding the first bonding component.

21. The expression plasmid according to claim 18, **characterised in that** it contains the nucleotide sequence of Fig. 8a for encoding the first bonding component.

22. The expression plasmid according to any of claim 18 to 21, **characterised in that** it contains the nucleotide sequence of Fig. 9a or Fig. 10a for encoding the second bonding component.

23. A bonding reagent for costimulating an effector cell, having a first bonding component for a cell surface protein and a second bonding component for a costimulatory acting molecule of an effector cell, **characterised in that** the first bonding component or/and the second bonding components have multivalent binding capabilities.

24. The bonding reagent according to claim 23, **characterised in that** either of the first bonding component and the second bonding component can by antibodies or portions thereof which have a binding domain.

25. The bonding reagent according to claim 23 or 24, **characterised in that** the cell suface protein is a virus protein, a growth factor receptor, an oncogene product, an adhesion molecule, streptavidin or an antibody.

26. The bonding reagent according to any one of claims 23 to 25, **characterised in that** the first bonding component is at least one single chain Fv fragment directed against the F protein of Newcastle Disease Virus on the cell surface of NDV infected cells or the first bonding component is at least one single chain Fv fragment comprising the peptide sequence of Fig. 8b.

27. The bonding reagent according to any one of claims 23 to 26, **characterised in that** the costimulatory acting molecule is a CD2, CD3, CD19, CD22, CD26, CD28, or CTLA-4 receptor.

28. The bonding reagent according to claim 27, **characterised in that** the second bonding component is at least one single chain Fᵥ fragment directed against the costimulatory acting molecule CD28 of effector cells and comprises the peptide sequence of Fig. 9b or Fig. 10b.

29. The bonding reagent according to any of claims 23 to 28, **characterised in that** the bonding components are linked with one another via a complex.

30. The bonding reagent according to claim 29, **characterised in that** the complex consists of streptavidin (avidin) and biotin.

31. The bonding reagent according to claim 29, **characterised in that** the complex consists of S protein and S peptide.

32. The bonding reagent according to any one of claims 23 to 28, **characterised in that** it is the bonding reagent according to any one of claims 9 to 14, comprising a plurality of first portions thereof or/and of second portions thereof.

33. A vaccine having inactivated cells, wherein one or more bonding reagent according to any of claims 1 to 14 or 28 to 32 are bound to a cell surface protein.

34. Use of a bonding reagent having a first bonding component for a cell surface protein and a second bonding component for a costimulatory acting molecule of an effector cell, for the production of a pharmaceutical composition for the treatment of tumor patients showing at least residual tumor cells.

35. Use according to claim 34, **characterised in that** it is the bonding reagent of any of claims 1 to 14 or 28 to 32.

36. Use according to claim 34 or 35, **characterised in that** the pharmaceutical composition comprises a tumor specifc virus, and the bonding reagent has a first bonding component with a bonding specifity for proteins of said tumor specific virus which are located on the cell surface of infected cells.
